# EUROPEAN PATENT SPECIFICATION

(11) **EP 3 369 829 B1**
(45) Date of publication and mention of the grant of the patent: **29.04.2020**
(21) Application number: 18159809.5
(22) Date of filing: 02.03.2018
(51) Int. Cl.: C12Q 1/6883

(54) **METHODS FOR DETECTING ATTENTION-DEFICIT/HYPERACTIVITY DISORDER**
VERFAHREN ZUR AUFMERKSAMKEITSDEFIZIT- UND HYPERAKTIVITÄTS-STÖRUNG (ADHS) DIAGNOSE
MÉTHODES POUR LA DÉTECTION DE TROUBLE DÉFICITAIRE DE L'ATTENTION AVEC HYPERACTIVITÉ (ADHD)

(30) Priority: 03.03.2017 US 201762466586 P
(43) Date of publication of application: 05.09.2018
(73) Proprietor: Kaohsiung Chang Gung Memorial Hospital, Kaohsiung City 83301 (TW)
(72) Inventor: WANG, Liang-Jen, Kaohsiung City 83301 (TW); LI, Sung-Chou, Kaohsiung City 83301 (TW); KUO, Ho-Chang, Kaohsiung City 83301 (TW)
(74) Representative: Cabinet Becker et Associés

(56) References cited:
- WO-A1-2013/116589
- WU LI HUI ET AL: "Circulating MicroRNA Let-7d in Attention-Deficit/Hyperactivity Disorder", NEUROMOLECULAR MEDICINE, HUMANA PRESS, US, vol. 17, no. 2, 28 February 2015 (2015-02-28), pages 137-146, XP035498027, ISSN: 1535-1084, DOI: 10.1007/S12017-015-8345-Y [retrieved on 2015-02-28]
- HASAN KANDEMIR ET AL: "Evaluation of several micro RNA (miRNA) levels in children and adolescents with attention deficit hyperactivity disorder", NEUROSCIENCE LETTERS, vol. 580, 12 August 2014 (2014-08-12), pages 158-162, XP055464250, AMSTERDAM, NL ISSN: 0304-3940, DOI: 10.1016/j.neulet.2014.07.060
- CHENG-TSUNG PAN ET AL: "miRSeq: A User-Friendly Standalone Toolkit for Sequencing Quality Evaluation and miRNA Profiling", BIOMED RESEARCH INTERNATIONAL, vol. 2014, 24 June 2014 (2014-06-24), pages 1-8, XP055464395, ISSN: 2314-6133, DOI: 10.1155/2014/462135

## Description

### BACKGROUND OF THE INVENTION

Attention-Deficit/Hyperactivity Disorder (ADHD) is a common childhood and adolescent psychiatric disorder, characterized inattention, hyperactivity and impulsivity. It affects roughly 3% to 10% of school-age children worldwide, more than half of the ADHD children continue to struggle with symptoms through adulthood. Early and accurate diagnosis of ADHD is crucial to ameliorate patient's functional impairments throughout the lifecycle.

The diagnosis of ADHD is based upon the observation of behavioral signs and verbal reports of patients or their family, which are prone to bias. There is continuing controversy regarding the reliability and validity ofADHD diagnosis. L.H. Wu et al. ("Circulating MicroRNA Let-7d in Attention-Deficit/Hyperactivity Disorder", Neuromol Med (2015) 17:137-146) identified a higher circulating level of miRNA Let-7d in ADHD subjects. H. Kandemir et al. ("Evaluation of several miRNA levels in children and adolescents with attention deficit hyperactivity disorder" Neuroscience Letter 580 (2014) 158-162) found miRNA 18a-5p, 22-3p, 106b-5p and 107 levels were lower in ADHD patients and miRNA 155a-5p level was higher in ADHD patients compared to the control. WO 2013/116589 discloses methods for the diagnosis and treatment of a mental disorder, such as attention-deficit hyperactivity disorder, by measuring the level of miR-25, miR-98 and miR-185. Cheng-Tsung Pan et al. ("miRSeq: A User-Friendly Standalone Toolkit for Sequencing Quality Evaluation and miRNA Profiling" Biomed Research International Vol 2014, 24 June 2014, 1-8) teaches the use of miRSeq to determine miRNA expression profiles.

There is an unmet need for the identification of biomarkers, particularly measurable in vivo and noninvasive, to accurately diagnose ADHD and facilitate the development of novel therapeutic strategies. The present invention satisfies this and other needs.

### BRIEF SUMMARY OF THE INVENTION

The present invention is directed to methods for detecting ADHD in a subject, comprising the step of measuring the expression level of at least the following miRNA in the test sample of the subject: miR140-3p (SEQ ID NO:1), wherein a lower expression level of said miRNA in the test sample, relative to the expression level of corresponding miR140-3p in an ADHD-free sample, is indicative of the subject having ADHD.

The method of the invention may further comprise the step of measuring the expression level of miRNA-151a-3p and/or miRNA-126-5p in the test sample.

The invention will become more apparent when read with the accompanying figures and detailed description which follow.

### BRIEF DESCRIPTION OF THE DRAWINGS

A more complete understanding of the invention may be obtained by reference to the accompanying drawings, when considered in conjunction with the subsequent detailed description. The embodiments illustrated in the drawings are intended only to exemplify the invention and should not be construed as limiting the invention to the illustrated embodiments.
FIG. 1 is a flow chart illustrating the study design of the Examples.
FIG. 2A- Fig. 2M are bar graphs illustrating the expression levels of 13 miRNAs (measured in threshold cycle, ΔCt) that are differentially expressed in ADHD and control subjects:, miR-140-3p (FIG 2A), let-7g-5p (FIG. 2B), miR-30e-5p (FIG. 2C), miR-223-3p (FIG. 2D), miR-142-5p (FIG. 2E), miR-486-5p (FIG. 2F), miR-151a-3p (FIG. 2G), miR-151a-5p (FIG. 2H), miR-126-5p (FIG. 2I), miR-27a-3p (FIG. 2J), miR-101-3p (FIG. 2K), miR-150-5p (FIG. 2L), and miR-92a-3p (FIG. 2M) in ADHD and control samples using qPCR analysis. "**" and "*" denote p<0.01 and p<0.05, respectively.
FIG. 3 Panels A-B are Receiver operating characteristic (ROC) curves of the ADHD diagnostic model (a support vector machines (SVM) classification model) for discriminating ADHD from the non-ADHD (control) subjects in a younger age group (Panel A) and an older age group (Panel B).

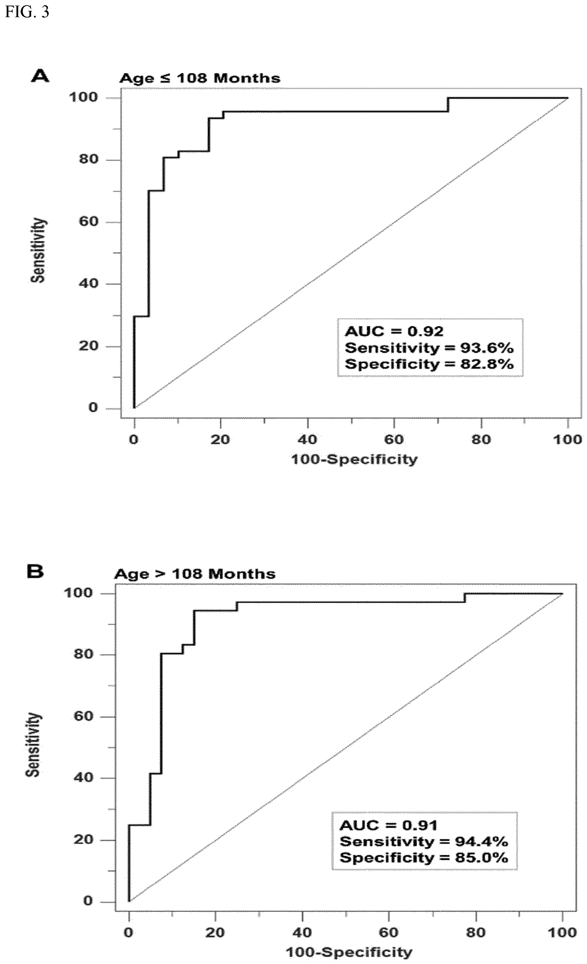

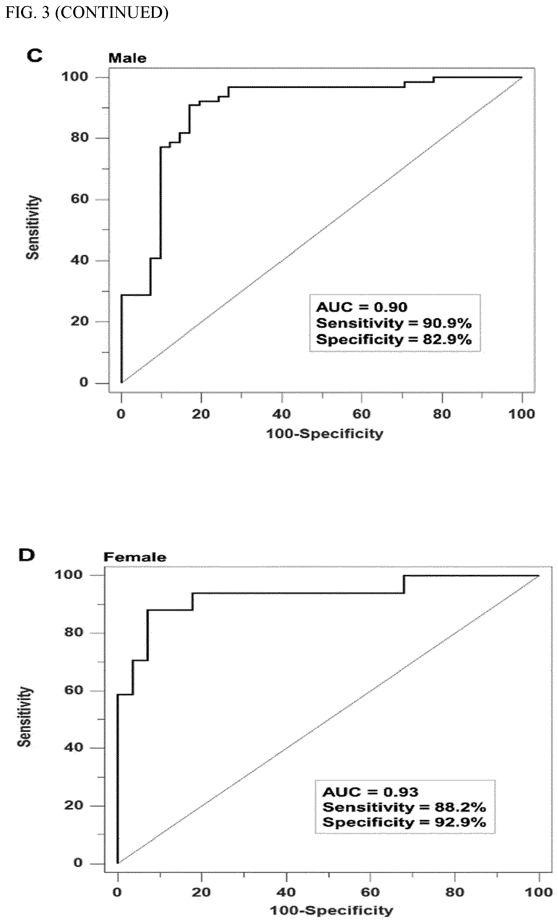

FIG. 3 Panels C and D are ROC curves of the ADHD diagnostic model to discriminate ADHD from the non-ADHD (control) in male (Panel C) and in female (Panel D).

### DETAILED DESCRIPTION OF THE INVENTION

As used herein, the articles "a" and "an" refer to one or more than one (*i.e.,* at least one) of the grammatical object of the article.

The use of the term "or" in the claims is used to mean "and/or" unless explicitly indicated to refer to alternatives only or the alternatives are mutually exclusive, although the disclosure supports a definition that refers to only alternatives and "and/or."

"Patient" or "subject" as used herein refers to a mammalian subject diagnosed with or suspected of having or developing ADHD. Exemplary subjects may be humans, apes, dogs, pigs, cattle, cats, horses, goats, sheep, rodents and other mammalians that can develop ADHD.

As used herein interchangeably, a "microRNA," "miR," or "miRNA" refers to the unprocessed (e.g., precursor) or processed (e.g., mature) RNAtranscript from a miR gene. MicroRNAs are endogenous non-coding single-stranded RNAs of approximately 22 nucleotides in length that negatively regulate gene expression in eukaryotes and constitute a novel class of gene regulators (Chua, et al. (2009) Curr. Opin. Mol. Ther. 11:189-199). Individual miRNAs have been identified and sequenced in different organisms, and they have been given names. Names of miRNAs and their sequences are provided herein.

All numbers herein may be understood as modified by "about." As used herein, the term "about," when referring to a measurable value such as a temporal duration and the like or a range, is meant to encompass variations of ± 10% from the specified value, as such variations are appropriate to expression of miRNA level unless otherwise specified.

### METHODS FOR DIAGNOSING ATTENTION-DEFICIT/HYPERACTIVITY DISORDER (ADHD)

The present invention is based, in part, on the identification of particular miRNA whose expression level is increased or decreased in a test sample of a subject, relative to the predetermined level of the corresponding miRNA in an ADHD-free sample. Some embodiments of the present invention are directed to methods of diagnosing whether a subject has, or is at risk for developing ADHD, comprising measuring the expression level of at least one miRNA in the test sample from the subject and comparing the expression level of the corresponding miRNA in the ADHD-free or control sample. In one embodiment, a higher miRNA expression level in the test sample, relative to that of the control sample, is indicative of the subject having ADHD. In another embodiment, a lower miRNA expression level in the test sample, relative to that of the control sample, is indicative of the subject having ADHD.

The predetermined miRNA expression level in an ADHD-free or control sample is from a representative pool of ADHD-free individuals, and are a mean, median or other statistically manipulated or otherwise summarized or aggregated representative miRNA expression level in the ADHD-free or control samples.

In one embodiment, the miRNA expression level is presented as the threshold cycles (Ct). Ct is defined as the polymerase chain reaction (PCR) cycle at which the fluorescent signal of the reporter dye crosses an arbitrarily placed threshold and is determined by known methods disclosed in Thomas D Schmittgen & Kenneth J Livak, "Analyzing real-time PCR data by the comparative CT method," Nature Protocols, Vol.3, No.6 (2008), 1101-1108 and Caifu Chen et al. "Real-time quantification of microRNAs by stem-loop RT-PCR" Nucleic Acids Research, 2005, Vol. 33, No. 20 e179. In another embodiment, the miRNA expression level is the quantitative miRNA expression measured by methods described herein or any method known in the art. In another embodiment, the miRNA of the ADHD patient is compared with the predetermined miRNA expression level of an ADHD-free or control sample using the machine learning network described herein.

A "higher" or a "lower" miRNA expression is a relative term and can be determined by comparison of the miRNA expression level in the test sample to that from a referenced pool of subjects known to be ADHD-free (i.e., control subjects). In some embodiments, the expression of miRNA level in a test sample is 1%, 5%, 10%, 15%, 20%, 25%, 30%, 35%, 40%, 45%, 50%, 55%, 60%, 65%, 70%, 75%, 80%, 85%, 90%, 95%, 100%, 150%, 200%, 250%, 300%, 350%, 400%, 450%, 500% or any % thereinbetween higher than the miR expression level from a referenced pool of ADHD-free subjects. In other embodiments, the expression of miRNA level in a test sample is 1%, 5%, 10%, 15%, 20%, 25%, 30%, 35%, 40%, 45%, 50%, 55%, 60%, 65%, 70%, 75%, 80%, 85%, 90%, 95%, 100%, 150%, 200%, 250%, 300%, 350%, 400%, 450%, 500% or any % thereinbetween lower than that from a referenced pool of ADHD-free subject.

In the invention, the expression level of at least miR-140-3p is measured in the test sample of a subject to diagnose ADHD. The method described herein may further comprise measuring the expression level of at least one of the following miRNAs:
- let-7g-5p, miR-486-5p, miR-151a-5p, , miR-126-5p, or miR-151a-3p;
- let-7g-5p, miR-486-5p, miR-151a-3p, miR-151a-5p, miR-126-5p, miR-30e-5p, miR-223-3p, or miR-142-5p; or
- let-7g-5p, miR-486-5p, miR-151a-3p, miR-151a-5p, miR-126-5p, miR-30e-5p, miR-223-3p, miR-142-5p, miR-27a-3p, miR-101-3p, miR-150-5p, or miR-92a-3p.

The expression level of miR-140-3p is measured in the test sample of a subject and a lower miR-140-3p level in the test sample relative to that of the control sample, is indicative of the subject having ADHD. In some embodiments, the measurement of two or more miRNA expression levels offer a higher accuracy, sensitivity or specificity for ADHD diagnosis.

Measuring the level of miRNA expression refers to quantifying the amount of miRNA present in a sample. Measuring the expression level of any miRNA can be achieved using methods described herein, such as by real-time PCR, Northern blot analysis, or any technique known to those of skill in the art. Measuring the expression level of miRNA includes measuring the expression of either a mature form of miRNA or a precursor form that is correlated with miRNA expression.

In a particular embodiment, the level of at least one miRNA is detected using Northern blot analysis. For example, total cellular RNA can be purified from cells by homogenization in the presence of nucleic acid extraction buffer, followed by centrifugation. Nucleic acids are precipitated, and DNA is removed by treatment with DNase and precipitation. The RNA molecules are then separated by gel electrophoresis on agarose gels according to standard techniques, and transferred to nitrocellulose filters. The RNA is then immobilized on the filters by heating. Detection and quantification of specific RNA is accomplished using appropriately labeled DNA or RNA probes complementary to the RNA in question. See, for example, Molecular Cloning: A Laboratory Manual, J. Sambrook et al., eds., 2nd edition, Cold Spring Harbor Laboratory Press, 1989, Chapter 7.

In some embodiments, use of a microarray is desirable. A microarray is a microscopic, ordered array of nucleic acids, proteins, small molecules, cells or other substances that enables parallel analysis of complex biochemical samples. Microarrays can be fabricated using a variety of technologies, including printing with fine-pointed pins onto glass slides, photolithography using pre-made masks, photolithography using dynamic micromirror devices, ink-jet printing, or electrochemistry on microelectrode arrays.

Microarray analysis of miRNAs, for example, can be accomplished according to any method known in the art. In one embodiment, RNA is extracted from a cell such as white blood cell or a sample, the small RNAs (18-26-nucleotide RNAs) are size-selected from total RNA using denaturing polyacrylamide gel electrophoresis. Oligonucleotide linkers are attached to the 5' and 3' ends of the small RNAs and the resulting ligation products are used as templates for an RT-PCR reaction with 10 cycles of amplification. The sense strand PCR primer has a fluorophore attached to its 5' end, thereby fluorescently labeling the sense strand of the PCR product. The PCR product is denatured and then hybridized to the microarray. An PCR product, referred to as the target nucleic acid that is complementary to the corresponding miRNA capture probe sequence on the array will hybridize, via base pairing, to the spot at which the capture probes are affixed. The spot will then fluoresce when excited using a microarray laser scanner. The fluorescence intensity of each spot is then evaluated in terms of the number of copies of a particular miRNA, using a number of positive and negative controls and array data normalization methods, which will result in assessment of the level of expression of a particular miRNA.

In one embodiment, the microarray comprises miRNA-specific probe oligonucleotides for sequencing or measuring the one or more of the following miRNAs: miR140-3p, let-7g-5p, miR-486-5p, miR-151a-3p, miR-151a-5p, miR-126-5p, miR-30e-5p, miR-223-3p, miR-142-5p, miR-27a-3p, miR-101-3p, miR-150-5p, miR-92a-3p, or the combination thereof.

In some embodiments, use of quantitative RT-PCR is desirable. Quantitative RT-PCR (qRT-PCR) is a modified PCR used to rapidly measure the quantity of a product of polymerase chain reaction. qRT-PCR is commonly used for the purpose of determining whether a genetic sequence, such as a miRNA, is present in a sample, and if it is present, the number of copies in the sample. Any method of PCR that can determine the expression of a nucleic acid molecule, including a miRNA, falls within the scope of the present disclosure. There are several variations of the qRT-PCR method known in the art, include, but are not limited to, via agarose gel electrophoresis, the use of SYBR Green (a double stranded DNA dye), and the use of a fluorescent reporter probe.

Some potential miRNAs biomarkers of ADHD, involved in unknown biological mechanisms can be identified using a global screening technology, such as microarray or next-generation sequencing (NGS). NGS is a technique of global screening for miRNA expression profile, showing high sensitivity and low background noise, and potentially discovers ADHD markers which have not been identified.

The identification of miRNAs that are differentially expressed in ADHD and non-ADHD subjects, allows the use of this information in a number of ways. For example, a particular treatment regime may be evaluated (e.g., to determine whether a therapy is effective in a subject with ADHD). Similarly, diagnosis may be done or confirmed by comparing the miRNA expression level in a test sample with known expression profiles from non-ADHD samples. Furthermore, these miRNA expression profiles allow screening of drug candidates that suppress miRNA expression in ADHD, or convert a poor prognosis profile to a better prognosis profile.

In one exemplary embodiment, methods for establishing ADHD-specific machine learning algorithms are provided, comprising: (a)determining the ΔCt value of at least miR140-3p from a reference pool of subjects without ADHD and a reference pool of subjects having ADHD; and (b) registering at least one ΔCt value of the miRNA from the reference pool of subjects without ADHD and at least one ΔCt value of the miRNA from the reference pool of subjects with ADHD from step (a) into a machine learning algorithm; and (c) establishing a binary classification model using the machine learning algorithm in step (b).

Non-limiting examples of machine learning network include Support Vector Machines (SVM), artificial neural networks, decision tree learning (e.g., CART, ID3, C4.5, CHAID, MARS, and Conditional Inference Trees), instance based learning (e.g., K-Nearest Neighbor), Bayesian networks, genetic algorithms and ensemble learning (e.g., Bagging and Boosting).

### KITS FOR DIAGNOSING ATTENTION-DEFICIT/HYPERACTIVITY DISORDER (ADHD)

It is herein described kits for use in diagnosing ADHD. In one exemplary embodiment, the kit comprises at least one agent for sequencing or measuring the expression level of one or more miRNAs selected from the group consisting of: miR140-3p, let-7g-5p, miR-486-5p, miR-151a-3p, miR-151a-5p, miR-126-5p, miR-30e-5p, miR-223-3p, miR-142-5p, miR-27a-3p, miR-101-3p, miR-150-5p and miR-92a-3p, in a test sample of a subject in need of a diagnosis of ADHD. In another embodiment, the kit comprises at least two agents for sequencing or measuring the expression level of at least two of the following miRNAs: miR-151a-3p, miR-151a-5p, miR-126-5p, miR-140-3p or miR-486-5p in a test sample of a subject in need of a diagnosis of ADHD.

Non-limiting examples of the test sample include body fluid (e.g. serum, blood, and cerebrospinal fluid), cells (e.g., white blood cells) and tissue (brain biopsy).

In one embodiment, the kit further comprises an instruction for use of the kit to diagnose ADHD. In another embodiment, the agent is RT-PCR. In another embodiment, the agent is a probe oligonucleotide specific for sequencing or measuring any of the SEQ ID NO: 1 to SEQ ID NO: 13 miRNAs. The agent can be an agent known in the art for measuring the expression level of SEQ ID NO: 1 to SEQ ID NO: 13 miRNA.

Embodiments of the present invention are illustrated by the following examples, which are not to be construed in any way as imposing limitations upon the scope thereof. During the studies described in the following examples, conventional procedures were followed, unless otherwise stated. Some of the procedures are described below for illustrative purpose.

### Description of Materials and Methods Used in the Examples

Study Participant: The research protocol was approved by the Institutional Review Board (IRB) at Kaohsiung Chang Gung Hospital in Taiwan. Eligible patients with ADHD were treated at the Child Psychiatry outpatient of Kaohsiung Chang Gung Children's Hospital in Taiwan and control (ADHD-free) subjects were recruited from the same hospital for this study.

The inclusion criteria for the study were (a) clinical diagnosis of ADHD by a senior pediatric psychiatrist based on the criteria of the Diagnostic and Statistical Manual of Mental Disorders, Fourth Edition, Text Revision (DSM-IV-TR) after structured interviews based on the Chinese version of the Schedule for Affective Disorders and Schizophrenia for School-Age Children, epidemiologic version (K-SADS-E); (b) age between 6 and 16 years; (c) Han Chinese ethnic origin; and (d) no prior ADHD medication. Patients were excluded if they had a major physical illness (such as genetic, metabolic, or infectious conditions) or a history of neuropsychiatric disease, such as intellectual disabilities, autism spectrum disorder, bipolar disorders, major depressive disorders, psychotic disorders, substance use disorders, epilepsy or severe head trauma.

The control subjects were children without ADHD of Han Chinese ethnic origin, between the age of 6 and 16 years old, and free of any major physical illnesses or aforementioned major neuropsychiatric diseases.

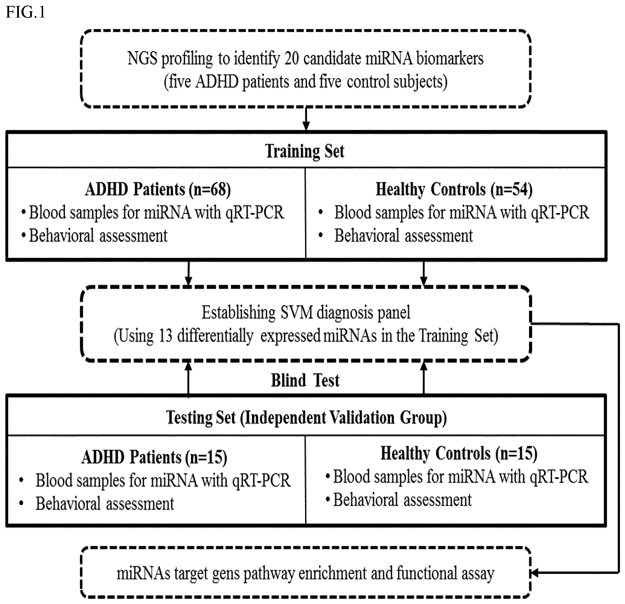

FIG.1 is a flow chart showing the study design. A preliminary study was conducted based on the blood samples from five ADHD patients and five control subjects and 20 miRNAs were identified as the potential biomarkers for ADHD using the NGS technique.

A Training Set based on the blood samples collected from 68 ADHD patients and 54 control subjects was used to establish a miRNA-based ADHD diagnostic model. A Testing Set based on the blood samples from 15 ADHD patients and 15 control subjects was used to further verify the miRNA-basedADHD diagnostic model identified in the Training Set.

### Collecting test samples and extracting RNA samples

Approximately 5 ml of blood was collected from each subject. The whole blood sample was centrifuged at 3000 rpm for 10 min, followed by the addition of a red blood cell (RBC) lysis buffer (RBC Bioscience, Taiwan) to remove the RBCs. The RBC-free sample was processed with the mirVana miRNA isolation kit (Life technology, USA) to extract total RNAs from the white blood cells (WBC). The integrity of the RNAs was determined using Agilent 2100 (Agilent Technologies, USA). RNA sample with an RNA integrity number (RIN) less than 8 was excluded.

### The evaluation of miRNAs profile in ADHD and ADHD-free samples

In the preliminary study, the RNA samples from five ADHD patients and five control subjects were evenly mixed to generate a pooled ADHD library and a pooled control library, respectively. The two pooled RNA libraries were prepared with TruSeq Small RNA preparation kit (Illumina, USA) and sequenced with MiSeq (Illumina), followed by miRSeq analysis to identify the 20 miRNA candidates for ADHD diagnosis.

### Real-time qPCR validation of miRNA

The real-time quantitative reverse transcription PCR (qRT-PCR) was used to determine the expression profiles of the 20 miRNAs in the blood samples of all of the enrolled subjects. The TaqMan MicroRNA Transcription Kit (Applied Biosystems, USA) was used to prepare the RNA samples. Reverse-transcription reactions were carried out on a Veriti 96 well thermal cycler (Applied Biosystems) in accordance with the manufacturer's instructions. The real-time PCR cycling condition was as follows: 95°C for 10 minutes, followed by 40 cycles at 95°C for 15s and 60°C for 1 min. miRNAs expression abundances were determined by the ΔCt values with the small nucleolar RNA U44 as the endogenous control.

### Clinical Measurements

All of the ADHD subjects and the control subjects were interviewed by a senior psychiatrist using the K-SADS-E diagnostic tool and ad child psychologist using the Wechsler Intelligence Scale for Children-Fourth Edition (WISC-IV), in a room designed to reduce variability in testing conditions. The Swanson, Nolan, and Pelham Version IV Scale (SNAP-IV) parent form and SNAP-IV teacher form were completed by the subject's parent(s) and the teacher, respectively. Subjects were interviewed by a clinician using the ADHD Rating Scale (ADHD-RS).

### Statistical Analysis

Data were analyzed using the statistical software package SPSS, version 16.0 (SPSS Inc., Chicago, IL, USA) and the MedCalc software Version 15.11.4. Variables were presented as either the mean (standard deviation) or frequency. Two-tailed *p* values of < 0.05 were considered statistically significant.

Library for Support Vector Machine (LIBSVM), an integrated software for support vector classification, regression and distribution estimation, was used to develop an ADHD specific SVM alignment model to discriminate control and ADHD subjects based on the miRNA expression. Receiver operating characteristic (ROC) curves and the area under curve (AUC) were used to evaluate both the specificity and sensitivity of the probability score yielded by the LIBSVM. The optimal diagnostic point of the signature was evaluated at the cut-off values of the probability score at 0.5. Pearson correlation was performed to analyze the relationships between the probability score and ADHD symptoms in ADHD patients.

### Results

### miRNA expression profile by NGS

The miRNA expression profile by NGS shows 18 candidate miRNAs were differentially expressed between ADHD patients and the control. The candidate miRNAs was defined as: (1) TPM (transcript per million) >1,000 in either ADHD group or control group; (2) Ratios of miRNAs of ADHD patients/control subjects were approximately higher than 1.50 or lower than 0.67. Based on previous research data, miR-181a-5p and let-7d were also considered as potential biomarkers. A total of 20 candidate miRNAs served as diagnostic biomarkers for ADHD: miR-29a-3p, miR-142-3p, miR-140-3p, miR-423-3p, miR-192-5p, miR-27a-3p, miR-101-3p, miR-150-5p, let-7g-5p, miR-30e-5p, miR-223-3p, miR-142-5p, miR-143-3p, miR-92a-3p, miR-486-5p, miR-151a-3p, miR-151a-5p, miR-126-5p, miR-181a-5p, and let-7d.

### qPCR validation

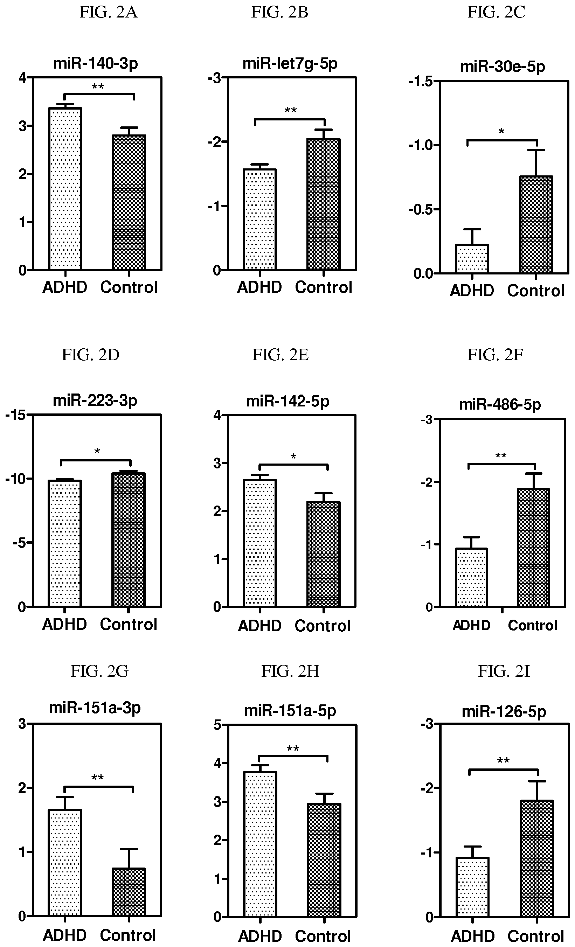

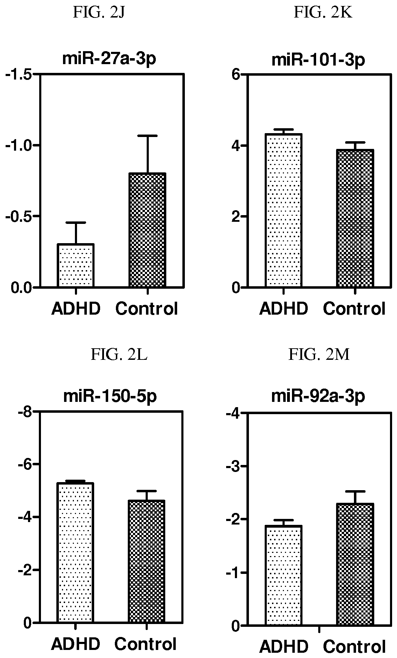

qPCR validation on all samples from the Training Set shows the expression levels of following 9 miRNAs were sinificantly different between ADHD and non-ADHD subjects in the Training Set: miR140-3p, let-7g-5p, miR-486-5p, miR-151a-3p, miR-151a-5p, miR-126-5p, miR-30e-5p, miR-223-3p, and miR-142-5p (FIG. 2A-FIG. 2I). FIG. 2J to FIG. 2M show the expression levels of miR-27a-3p, miR-101-3p, miR-150-5p, and miR-92a-3p were different between ADHD and non-ADHD subjects in the Training Set (0.1>P>0.05).

### The ADHD diagnostic model

To derive a more robust diagnosis model, the combination of the expression levels of the following 13 miRNAs was used to develop the ADHD diagnostic model using the SVM algorithm (hereafter "the ADHD diagnostic model"): miR-140-3p, miR-27a-3p, miR-101-3p, miR-150-5p, let-7g-5p, miR-30e-5p, miR-223-3p, miR-142-5p, miR-92a-3p, miR-486-5p, miR-151a-3p, miR-151a-5p, and miR-126-5p. The Multivariate analysis of covariance confirmed that the expression of these 13 miRNAs was not confounded by age, sex or intelligence quotient. To derive the best discriminative validity, 5-fold cross validations was used to derive the optimal parameters, gamma=0.015625 and cost=256. Using the default probability cutoff of 0.5, the ADHD diagnostic model has a sensitivity of 86.8% and a specificity of 88.9%, leading to an area under curve (AUC) value of 0.94 (*p* < 0.001), when compare with the ADHD diagnosis by physician (the gold standard).

In addition, the combination of specific miRNAs lead to unexpected synergy in ADHD diagnostic accuracy (AUC), as shown in Table 1.

**Table 1. The AUC of miRNAs used in ADHD diagnosis.**

| miRNA | AUC |
|---|---|
| miR-140-3p | 0.68 |
| miR-486-5p | 0.8010 |
| miR-151a-3p | 0.6215 |
| miR-151a-5p | 0.7785 |
| miR-126-5p | 0.6206 |
| miR-140-3p+ miR-151a-3p | 0.8164 |
| miR-140-3p+ miR-126-5p | 0.9520 |
| miR-486-5p+ miR-151a-5p | 0.8461 |
| miR-486-5p+ miR-126-5p | 0.8668 |
| miR-151a-3p+ miR-151a-5p | 0.8413 |
| miR-151a-3p+ miR-126-5p | 0.6786 |

An independent blind test was conducted on 15 ADHD and 15 control subjects in the Testing Set. There was no significant difference between ADHD patients and control subjects in age, sex or intelligence quotient. The 13 miRNA ΔCt of the 30 subjects in the Testing Set was analysed using the ADHD diagnostic model. 13 of the 15 ADHD samples were correctly diagnosed with ADHD and 11 of the 15 control subjects were correctly classified as non-ADHD, demonstrating a sensitivity of 86.7%, a specificity of 73.3%, and an accuracy of 80%.

### Further validation of ADHD diagnostic model

The subjects in the Training Set and Testing Set were pooled together for additional sensitivity analysis. The subjects were stratified into a younger group (≤108 months) and an older group (>108 months) and the ROC analysis showed the probability scores from the ADHD diagnostic model effectively discriminate ADHD and non-ADND subjects in both age groups (FIG. 3A, AUC: 0.92, *p* < 0.001 and FIG. 3B, AUC: 0.91, *p* < 0.001). In addition, the subjects were stratified into male and female and the ROC analysis showed the probability scores from the ADHD diagnostic model effectively discriminate ADHD and non-ADND subjects in male (FIG. 3C, AUC: 0.90, *p* < 0.001) and female (Fig. 3D, AUC: 0.93, *p* < 0.001). Pearson correlation showed the probability score from the ADHDdiagnostic model positively correlated to the inattentive symptoms rated by parents (*r* = 0.485, *p* < 0.001), teachers (*r* = 0.507, *p* < 0.001) or clinicians (*r* = 0.632, *p* < 0.001), and hyperactive/impulsive symptoms rated by parents (*r* = 0.473, *p* < 0.001), teachers (*r* = 0.380, *p* < 0.001) or clinicians (*r* = 0.613, *p* < 0.001).

Several miRNAs, such as miR124-1 and let-7d, have been reported to be potentially associated with ADHD. Theses miRNAs were identified as they play a role in the neurobiology mechanisms of ADHD, based on literature review or the miRNA database. The 13 miRNAs used to construct the ADHD diagnostic model as described herein have not been reported to be involved in pathophysiology of ADHD and were identified by global screening technology (NGS).
SEQ ID NO:1 (hsa-miR-140-3p)
   UACCACAGGGUAGAACCACGG
SEQ ID NO:2 (hsa-let-7g-5p)
   UGAGGUAGUAGUUUGUACAGUU
SEQ ID NO:3 (hsa-miR-486-5p)
   UCCUGUACUGAGCUGCCCCGAG
SEQ ID NO:4 (hsa-miR-151a-3p)
   CUAGACUGAAGCUCCUUGAGG
SEQ ID NO:5 (hsa-miR-151a-5p)
   UCGAGGAGCUCACAGUCUAGU
SEQ ID NO:6 (hsa-miR-126-5p)
   CAUUAUUACUUUUGGUACGCG
SEQ ID NO:7 (hsa-miR-30e-5p)
   UGUAAACAUCCUUGACUGGAAG
SEQ ID NO:8 hsa-miR-223-3p
   UGUCAGUUUGUCAAAUACCCCA
SEQ ID NO:9 (hsa-miR-142-5p)
   CAUAAAGUAGAAAGCACUACU
SEQ ID NO:10 (miR-27a-3p)
   uucacaguggcuaaguuccgc
SEQ ID NO:11 (miR-101-3p)
   uacaguacugugauaacugaa
SEQ ID NO:12 (miR-150-5p)
   ucucccaacccuuguaccagug
SEQ ID NO:13 (miR-92a-3p)
   uauugcacuugucccggccugu

## Claims

1. An *in vitro* method for detecting attention deficit hyperactivity disorder (ADHD) in a subject, comprising the step of measuring the expression level of miR-140-3p in the test sample of the subject;
wherein a lower expression level of miR140-3p in the test sample, relative to the expression level of miR140-3p in an ADHD-free sample, is indicative of the subject having ADHD.

2. The method of claim 1, further comprising the step of measuring the expression level of miRNA-151a-3p in the test sample.

3. The method of claim 1 or 2, further comprising the step of measuring the expression level of miRNA-126-5p in the test sample.

4. The method of any of claims 1 to 3, wherein the miRNA expression level is determined by real-time PCR or by a probe oligonucleotide specific for said miRNA.

## Patentansprüche

1. Ein *in vitro* Verfahren zum Nachweis von Aufmerksamkeitsdefizit-/Hyperaktivitätsstörung (ADHD) in einer Person, umfassend den Schritt der Messung des Expressionsniveaus von miR-140-3p in einer Testprobe der Person;
wobei ein niedriges Expressionsniveau von miR140-3p in der Testprobe bezogen auf das Expressionsniveau von miR140-3p in einer ADHD-freien Probe anzeigt, dass die Person ADHD hat.

2. Das Verfahren nach Anspruch 1, außerdem umfassend den Schritt der Messung des Expressionsniveaus von miRNA-151a-3p in der Testprobe.

3. Das Verfahren nach Anspruch 1 oder 2, außerdem umfassend den Schritt der Messung des Expressionsniveaus von miRNA-126-5p in einer Testprobe.

4. Das Verfahren nach einem der Ansprüche 1 bis 3, wobei das miRNA-Expressionsniveau mittels Echtzeit-PCR oder mittels eines für besagte miRNA spezifischen Sonden-Oligonukleotids bestimmt wird.

## Revendications

1. Méthode *in vitro* pour détecter un trouble déficitaire de l'attention avec hyperactivité (TDAH) chez un sujet, comprenant l'étape de mesure du niveau d'expression de miR-140-3p dans l'échantillon de test du sujet ;
dans laquelle un niveau d'expression de miR140-3p dans l'échantillon de test plus faible que le niveau d'expression de miR140-3p dans un échantillon sans TDAH est indicatif du fait que le sujet présente un TDAH

2. Méthode selon la revendication 1, comprenant en outre l'étape de mesure du niveau d'expression d'ARNmi-151a-3p dans l'échantillon de test.

3. Méthode selon la revendication 1 ou 2, comprenant en outre l'étape de mesure du niveau d'expression d'ARNmi-126-5p dans l'échantillon de test.

4. Méthode selon l'une quelconque des revendications 1 à 3, dans laquelle le niveau d'expression d'ARNmi est déterminé par PCR en temps réel ou par une sonde oligonucléotidique spécifique dudit ARNmi.


### SEQUENCE LISTING

<110> KAOHSIUNG CHANG GUNG MEMORIAL HOSPITAL
<120> METHODS AND KITS FOR DETECTNG ATTENTION-DEFICIT/HYPERACTIVITY DISORDER
<130> B2685EP
<150> US 62/466,586
   <151> 2017-03-03
<160> 13
<170> PatentIn version 3.5
<210> 1
   <211> 21
   <212> RNA
   <213> Artificial Sequence
<220>
   <223> hsa-miR-140-3p
<400> 1
   uaccacaggg uagaaccacg g 21
<210> 2
   <211> 22
   <212> RNA
   <213> Artificial Sequence
<220>
   <223> hsa-let-7g-5p
<400> 2
   ugagguagua guuuguacag uu 22
<210> 3
   <211> 22
   <212> RNA
   <213> Artificial Sequence
<220>
   <223> hsa-miR-486-5p
<400> 3
   uccuguacug agcugccccg ag 22
<210> 4
   <211> 21
   <212> RNA
   <213> Artificial Sequence
<220>
   <223> hsa-miR-151a-3p
<400> 4
   cuagacugaa gcuccuugag g 21
<210> 5
   <211> 21
   <212> RNA
   <213> Artificial Sequence
<220>
   <223> hsa-miR-151a-5p
<400> 5
   ucgaggagcu cacagucuag u 21
<210> 6
   <211> 21
   <212> RNA
   <213> Artificial Sequence
<220>
   <223> hsa-miR-126-5p
<400> 6
   cauuauuacu uuugguacgc g 21
<210> 7
   <211> 22
   <212> RNA
   <213> Artificial Sequence
<220>
   <223> hsa-miR-30e-5p
<400> 7
   uguaaacauc cuugacugga ag 22
<210> 8
   <211> 22
   <212> RNA
   <213> Artificial Sequence
<220>
   <223> hsa-miR-223-3p
<400> 8
   ugucaguuug ucaaauaccc ca 22
<210> 9
   <211> 21
   <212> RNA
   <213> Artificial Sequence
<220>
   <223> hsa-miR-142-5p
<400> 9
   cauaaaguag aaagcacuac u 21
<210> 10
   <211> 21
   <212> RNA
   <213> Artificial Sequence
<220>
   <223> miR-27a-3p
<400> 10
   uucacagugg cuaaguuccg c 21
<210> 11
   <211> 21
   <212> RNA
   <213> Artificial Sequence
<220>
   <223> miR-101-3p
<400> 11
   uacaguacug ugauaacuga a 21
<210> 12
   <211> 22
   <212> RNA
   <213> Artificial Sequence
<220>
   <223> miR-150-5p
<400> 12
   ucucccaacc cuuguaccag ug 22
<210> 13
   <211> 22
   <212> RNA
   <213> Artificial Sequence
<220>
   <223> miR-92a-3p
<400> 13
   uauugcacuu gucccggccu gu 22